# EUROPÄISCHE PATENTSCHRIFT

(11) **EP 0 619 737 B1**
(45) Veröffentlichungstag und Bekanntmachung des Hinweises auf die Patenterteilung: **17.03.1999**
(21) Anmeldenummer: 93901754.7
(22) Anmeldetag: 24.12.1992
(51) Int. Cl.: A61K 31/725, A61K 31/73

(54) **PHARMAZEUTISCHE ZUSAMMENSETZUNG ZUR WUND-, NARBEN- UND KELOIDBEHANDLUNG**
PHARMACEUTICAL COMPOSITION FOR THE TREATMENT OF WOUNDS, SCARS AND KELOIDS
COMPOSITION PHARMACEUTIQUE POUR LE TRAITEMENT DE PLAIES, CICATRICES ET CHELOIDES

(30) Priorität: 03.01.1992 DE 4200080
(43) Veröffentlichungstag der Anmeldung: 19.10.1994
(73) Patentinhaber: Reinmüller, Johannes, Dr. med., W-65189 Wiesbaden (DE)
(72) Erfinder: Reinmüller, Johannes, Dr. med., W-65189 Wiesbaden (DE)
(74) Vertreter: Huber, Bernhard, Dipl.-Chem.
(86) Internationale Anmeldenummer: EP9202990
(87) Internationale Veröffentlichungsnummer: WO9312801

(56) Entgegenhaltungen:
- WO-A-89/04172
- GB-A- 2 129 300
- US-A- 4 605 691
- BIOMATERIALS, Band 12, Nr. 3, 1991; G. BIAGINI et al., Seiten 281-286 G. BIAGINI ET AL 'WOUND MANAGEMENT WITH N-CARBOXYBUTYLCHITOSAN.'
- CLINICAL MATERIALS, Band 8, Nr. 1-2, 1991; J.M. DAVIDSON et al., Seiten 171-177 J.M. DAVIDSON ET AL 'HYALURONATE DERIVATIVES AND THEIR APPLICATION TO WOUND HEALING: PRELIMINARY OBSERVATIONS.'
- AESTETHIC PLASTIC SURGERY, Band 11, Nr. 1, 1987; J.-P.A. NICOLAI et al., Seiten 29-32 J.-P.A. NICOLAI ET AL 'A PROTOCOL FOR THE TREATMENT OF HYPERTROPHIC SCARS AND KELOIDS'
- RIVISTA ITALIANA DI CHIRURGIA PLASTICA, Band 18, Nr. 2, 1986; A. SORRONE et al., Seiten 205-207 A. SORRONE ET AL 'NOSTRA ESPERIENZA SULL ' USO DELL' ACIDO IALURONICO (HA) IN CHIRURGIA TORACICA.'
- CLINICS IN PODIATRIC MEDICINE & SURGERY, Band 3, Nr. 3, Juli 1986; G.L. DOCKERY et al., Seiten 473-485 G.L. DOCKERY ET AL 'INTRALESIONAL INJECTIONS'
- ZEITSCHRIFT FüR ALLGEMEINMEDIZIN, Band 61, Nr. 30, 31 Oktober 1985, Seiten 1101-1103 H. FRIEDRICH 'HYPERTROPHE NARBEN UND KELOIDE'

## Beschreibung

Die Erfindung betrifft eine pharmazeutische Zusammensetzung zur Wund-, Narben- und Keloidbehandlung.

Bei vielen Menschen treten im Verlauf der Heilung von Hautwunden überschießende Neubildungen von Narbengewebe auf. Diese Gewebsneubildungen werden im medizinischen Sprachgebrauch als Narbenhypertrophie, oder in gravierenden Fällen als Keloid bezeichnet. Es handelt sich dabei um veranlagungsmäßig bedingte, unvorhersehbare Reaktionen bei der Wundheilung. Die Ursachen für eine überschießende Narbenbildung sind bisher nicht bekannt. Zur Zeit existiert daher auch kein wissenschaftlich begründetes kausales Behandlungsprinzip.

Zur Keloidbehandlung werden zahlreiche Methoden beschrieben (vgl. z.B. McCarthy/Convers "Plastic Surgery", Band 1, Seiten 732-747, W.B. Saunders, 1990). Sie basieren aber alle mehr oder weniger auf Empirie und sind im strengen wissenschaftlichen Sinne nicht abgesichert. Dies liegt einerseits am mangelnden Grundlagenwissen, aber auch daran, daß in der Praxis oft eine spontane Rückbildungstendenz unbehandelter Keloide beobachtet wird.

Daraus ergibt sich die überaus große Zahl von konkurrierenden Behandlungsmethoden, deren Wirksamkeit aber mit einem Zweifel behaftet ist, und von denen die wichtigsten nachfolgend kurz beschrieben werden.

Es ist zu unterscheiden zwischen mechanischen (physikalischen) und medikamentösen (chemischen) Behandlungsmethoden.

Die mechanischen Behandlungsmethoden umfassen die Anwendung von Druck auf das Keloid durch äußere Kompressionsverbände, und die Applikation von Folien aus Siliconelastomeren. Sehr nachteilig dabei ist es, daß die Anwendung über Monate und Jahre erfolgen muß. Sie ist auch nicht in allen Körperregionen gleichermaßen einsetzbar; so ist z.B. am Hals eine Kompression in ausreichendem Maße nicht zu erreichen. Ausgedehnte Kompressionsverbände bedeuten außerdem für den Träger stets eine erhebliche Einschränkung der Bewegungsfreiheit und Lebensqualität. Darüber hinaus schaffen sie auch erhebliche hygienische Probleme, insbesondere in der warmen Jahreszeit oder in warmen Ländern. Durch die mechanische Beanspruchung der verletzlichen Narbenhaut können Kompressionsverbände auch wieder zu erneuten Gewebsläsionen führen und die Keloidbildung damit erneut fördern.

Zu den physikalischen Methoden zählen weiter auch die Kältebehandlung (Kryotherapie) und die Lasertherapie. Beide Methoden erzeugen Gewebsnekrosen durch Kälte oder Hitze, wodurch die aktiven Bindegewebszellen und die Fasersubstanz innerhalb der Keloide zerstört werden. Die Keloide kollabieren vorübergehend, und es entstehen Gewebsdefekte. Bei der nachfolgenden Heilung dieser Defekte wandern erneut Bindegewebszellen ein, wodurch die Keloidbildung erneut einsetzt. Deshalb können mit diesem Behandlungsverfahren also immer nur kurzfristige Besserungen, aber keine endgültige Ausheilung erreicht werden.

Bei den medikamentösen Methoden muß zwischen äußerlich anzuwendenden und injizierbaren Zubereitungen unterschieden werden.

Bei den äußerlich anzuwendenden Mitteln handelt es sich zumeist um Salben mit den unterschiedlichsten Wirkstoffen, wie z.B. Hormone, Aminosäuren, Mucopolysaccharide. Auch Pflanzenextrakte, z.B. aus Stechapfel, werden angewendet. Ein bevorzugter Inhaltsstoff von Zubereitungen zur äußerlichen Behandlung von Narben und Narbenkeloiden ist das Heparin (vgl. z.B. Pharmazie in unserer Zeit 10 (1981), Seite 168 bis 181).

Allen äußerlich anzuwendenden Wirkstoffzubereitungen haftet der gleiche Nachteil an, nämlich daß die äußerliche Anwendung, d.h. das Auftragen auf die Haut über dem Keloid, keine ausreichenden Gewebsspiegel im Keloid selbst, das eine Dicke bis zu mehreren Zentimetern erreichen kann, gewährleistet. Durch die übermäßig gesteigerte Durchblutung innerhalb der Keloide werden die Wirkstoffe, sofern diese überhaupt die Hautbarriere durchdringen können, über den Blut- und Lymphstrom rasch abtransportiert. Die Diffusion der Wirkstoffe im Gewebe spielt deshalb bei der Verteilung nur eine untergeordnete Rolle.

Für den Wirkstoff Heparin ist es außerdem bekannt, daß er die epidermalen Schichten nicht durchdringen kann. Damit ist die Wirkung extern anzuwendender Zubereitungen, wie z.B. Salben auf der Basis von Heparin, auf die Bindegewebszellen zweifelhaft. Es ist deshalb nicht auszuschließen, daß die mit Heparin-haltigen Narbensalben erzielten Erfolge der Spontanheilung zuzuschreiben sind.

Bei den invasiven Behandlungsverfahren für Keloide werden Abkömmlinge des Hormons Cortisol (Hydrocortison) eingesetzt; wegen der bekannten gesteigerten Durchblutung innerhalb der Keloide und des damit verbundenen schnellen Abtransportes von Pharmaka werden dazu vorzugsweise die kristallinen, schwerlöslichen Formen verwendet, mit denen ein Depoteffekt erreicht werden kann. Da Cortisol und seine Abkömmlinge die Zellen des Bindegewebes stark hemmen, sind gegenwärtig mit der invasiven Verabreichung (Injektion) pharmazeutischer Zubereitungen auf der Basis kristalliner Corticoide (Corticosteoride) die quantitativ und qualitativ stärksten Veränderungen bei Keloiden zu erzielen.

Ein entscheidender Nachteil dieser Therapie mit Corticoiden ist jedoch die fehlende Steuerbarkeit der Reaktion. Daher kann es bei der praktischen Anwendung entweder zu einer unerwünschten Narbenatrophie, d.h. zu einem Auseinanderweichen und Einsinken der Narbe mit entsprechender Entstellung kommen, oder zu einer zu geringen Reaktion des Gewebes mit mangelhaftem Behandlungserfolg.

Ein weiterer Nachteil der Corticoidtherapie ist in der systemischen Wirkung des gelösten Wirkstoffes zu sehen, wodurch sich die Anwendung bei großflächigen Keloiden oder bei Kindern verbietet.

Aufgabe der Erfindung ist die Bereitstellung einer pharmazeutischen Zusammensetzung zur Wund-, Narben- und Keloidbehandlung, mit der die vorstehend bei bisherigen Behandlungsweisen auftretenden Nachteile, insbesondere die unerwünschten Begleiterscheinungen einer Corticoidtherapie, wie z.B. lokale Überreaktion, vermieden werden können, die leicht und erfolgreich appliziert werden kann, und die vom Organismus abgebaut wird.

Diese Aufgabe wird mit der erfindungsgemäßen pharmazeutischen Zusammensetzung nach Patentanspruch 1 gelöst, die vernetzte Glykosaminglykane und übliche pharmazeutische Hilfs- und/oder Trägerstoffe enthält.

Zweckmäßige Ausgestaltungen davon sind Gegenstand der Ansprüche 2 bis 15.

Weiterer Gegenstand ist auch ein Verfahren gemäß Anspruch 16 zur Herstellung der erfindungsgemäßen pharmazeutischen Zusammensetzungen.

Mit den erfindungsgemäßen Zusammensetzungen ist es möglich, die unerwünschten Eigenschaften der Corticoidtherapie zu vermeiden. Es wurde nämlich gefunden, daß die erfindungsgemäß eingesetzten vernetzten Glykosaminglykane (GAG, Mucopolysaccharide) hemmend auf die Keloidbildung wirken, wenn sie nicht-topisch (intraläsional) appliziert werden. Dabei treten keine lokalen Überreaktionen wie bei den Corticoiden auf, und ebenso keine systemischen Wirkungen. Ein weiterer Vorteil ist die biologische Abbaubarkeit im Organismus.

Darüber hinaus lassen sich mit den erfindungsgemäßen Zusammensetzungen auch tiefe Narbenbildungen im Bindegewebe, wie z.B. die Dupuytren'sche Erkrankung der Handflächen oder die sogenannte Induratio penis plastica (IPP), die ohne vorausgegangene Verletzung (Kontinuitätstrennung) entstehen, erfolgreich behandeln.

Unter erfindungsgemäß vernetzten Glykosaminglykanen sind solche zu verstehen, in denen 2 oder mehrere gleiche und/oder verschiedene Glykosaminglykane miteinander unter Bildung einer molekularen Einheit verbunden sind. Vorzugsweise erfolgt die Vernetzung durch Chelatbildung, Komplexbildung und/oder Salzbildung und insbesondere durch eine chemische Vernetzung.

Als Glykosaminglykane können natürliche oder synthetische Glykosaminglykane, oder auch damit chemisch verwandte Substanzen eingesetzt werden. Sie können anionischen oder auch kationischen Charakter besitzen.

Als natürliche Glykosaminglykane werden vorzugsweise solche eingesetzt, die im menschlichen Bindegewebe vorkommen, insbesondere z.B. Hyaluronsäure, Heparin, Heparinsulfat, Chondroitinsulfat. Als synthetische Glykosaminglykane werden vorzugsweise sulfatierte Polysaccharide eingesetzt. Mit Glykosaminglykanen chemisch verwandte, erfindungsgemäß eingesetzte Substanzen sind vorzugsweise solche biologischen Ursprungs und insbesondere Chitosamin und Chitosan oder deren Derivate, wie z.B. N-Carboxybutylchitosan (vgl. R. Muzzarelli et al., Carbohydrate Polymers 11 (1989) 307-320). Die Bildung von Chelaten, schwerlöslicher Salze oder Komplexe der Glykosaminglykane, die ebenfalls eine Vernetzung im erfindungsgemäßen Sinne darstellt, erfolgt durch einen Zusammenhalt durch ionische Kräfte, wie sie für derartige Chelate, Salze oder Komplexe üblich ist.

Die Herstellung der erfindungsgemäß verwendeten vernetzten Glykosaminglykane kann auf an sich bekannte Weise erfolgen. Die chemische Vernetzung erfolgt dabei im allgemeinen durch Vernetzung mit bifunktionellen reaktiven Agentien, wie z.B. Glutaraldehyd oder Carbodiimid; über bifunktionelle Aminosäuren, z.B. Lysin, Protamine oder Albumine, können z.B. aber auch Vernetzungen über Amidbindungen hergestellt werden. Werden synthetisch hergestellte Glykosaminglykane oder Analoge davon erfindungsgemäß eingesetzt, so können diese bereits bei der Herstellung primär vernetzt werden, so daß eine weitere Behandlung zur Vernetzung nicht erforderlich ist. Zum Teil sind derartige Glykosaminglykane bereits im vernetzten Zustand handelsüblich erhältlich und können dann direkt erfindungsgemäß eingesetzt werden (z.B. "Hylon" und "Hylagel", eine vernetzte Hyaluronsäure der Fa. Biomatrix, NJ, USA; zur Herstellung vgl. auch US-A-4713448, US-A-4605691).

Vorzugsweise werden identische Glykosaminglykane vernetzt und dann erfindungsgemäß eingesetzt, aber auch die Kombination von zwei oder mehreren verschiedenen oder zum Teil verschiedenen Glykosaminglykanen ist erfindungsgemäß möglich. In einer besonders bevorzugten Ausführungsform werden extrem langkettige Glykosaminglykane (Molekulargewicht vorzugsweise zwischen 100000 bis 1000000) eingesetzt; dabei kann der Vernetzungsgrad dann gering bleiben. Die Produkte sind praktisch proteinfrei.

In einer besonders bevorzugten Ausführungsform der Erfindung wird als vernetztes Glykosaminglykan intermolekular vernetztes Heparin eingesetzt.

Die intermolekulare Vernetzung von Heparin kann z.B. auf folgende Weise durchgeführt werden: Handelsüblich erhältliches Heparin wird mit verdünnter Salpetersäure behandelt, wodurch am Heparin reaktive Aldehydgruppen ausgebildet werden; danach erfolgt eine reduktive Animierung mittels NaBH₃ CN. Über die freien Aminofunktionen des Heparins werden kovalente Bindungen zwischen den einzelnen Heparinketten (End- und Seitenketten) ausgebildet.

Eine Vernetzung des Heparins durch Komplexbildung erfolgt vorzugsweise mit Protaminen, Gentamycin oder Vancomycin, Metallionen wie z.B. Fe²⁺, Zn²⁺.

Die erfindungsgemäßen pharmazeutischen Zusammensetzungen enthalten die vernetzten Glykosaminglykane vorzugsweise in Mengen von 0,1 bis 20 Gew.-%, bezogen auf die gesamte pharmazeutische Zusammensetzung, insbesondere in einer Menge von 0,5 bis 10 Gew.-% und besonders bevorzugt in einer Menge von 1 bis 5 Gew.-%.

Die erfindungsgemäßen pharmazeutischen Zusammensetzungen können in Form von intraläsional applizierbaren Zubereitungen vorliegen und insbesondere in Form injizierbarer Gele, vorzugsweise mit einem Wassergehalt von 80 bis 99 Gew.-%, oder aber auch als wasserfreie Vorstufe, z.B. lyophilisiertes Pulver in Form eines Puders. Als pharmazeutische Hilfs- und Trägerstoffe können hierfür übliche, für die erfindungsgemäße Anwendung geeignete und mit den vernetzten Glykosaminglykanen kompatible Stoffe eingesetzt werden. Der bevorzugte Trägerstoff ist Wasser.

Als pharmazeutische Hilfsstoffe können die erfindungsgemäßen pharmazeutischen Zusammensetzungen, z.B. Mittel zur pH-Wert-Einstellung, Stabilisierungsmittel, Antioxidantien, Lösungsvermittler, penetrationsfördernde Mittel, Konservierungsmittel und/oder Gelbildner enthalten, wie sie in derartigen Zusammensetzungen üblicherweise verwendet werden. Sie werden in den in derartigen Zubereitungen üblichen Mengen verwendet.

Die erfindungsgemäßen pharmazeutischen Zusammensetzungen können gegebenenfalls neben den eigentlichen Wirkstoffen (vernetzte Glykosaminglykane) auch noch weitere pharmazeutische Wirkstoffe, die mit den vernetzten Glykosaminglykanen im Rahmen der Applikation verträglich sind, enthalten, z.B. Wirkstoffe zur Therapie von Hauterkrankungen (Dermatosen), Antibiotika (insbesondere Antibiotika mit gegenpoliger Ladung, wie z.B. Gentamycin und Vancomycin mit positiver Ladung für z.B. vernetztes Heparin, oder z.B. Penicilline oder Cephalosporine mit negativer Ladung für z.B. vernetztes Chitosamin), Sulfonamide, Desinfektionsmittel, Hormone (z.B. Corticoide) und Hormonabkömmlinge (z.B. Cortisol), lokale Anästhetika, z.B. vom Typ des Lidokains oder Novokains, vasoaktive Substanzen zur Gefäßkonstriktion (Vermeidung von Blutungen), Adrenalin, Enzyme wie z.B. Hyaluronidase, Interleukine, Wachstumsfaktoren (growth factor) z.B. EGF, PDGF und/oder IGF, Hautpflegemittel und/oder durchblutungsfördernde (hyperämisierende) Mittel. Diese Stoffe können, wie z.B. Antibiotika mit gegenpoliger Ladung, an die Glykosaminglykane fest gebunden vorliegen, also als Bestandteil der vernetzten Glykosaminglykane, und werden dann mit dem Abbau der vernetzten Glykosaminglykane freigesetzt, oder aber sie können nach Art eines "controlled release"-Systems freigesetzt werden.

Bei der erfindungsgemäß bevorzugten Applikation in Form einer Injektion können die Zubereitungen z.B. zur Vermeidung von Schmerzen beim Einstich mit der Injektionskanüle Lokalanästhetika enthalten. Als Antibiotikum enthalten die Zubereitungen vorzugsweise ein anionisch oder kationisch geladenes Molekül, z.B. Gentamycin, das gegenionisch an die vernetzten Glykosaminglykane gebunden wird und damit in loco fixiert bleibt, wodurch die Wirkung entsprechend verlängert wird.

Die Herstellung der erfindungsgemäßen Zusammensetzungen kann auf eine für die Herstellung derartiger Zusammensetzungen an sich übliche, allgemein bekannte Weise erfolgen. Dabei ist die Reihenfolge der Vermischung der einzelnen Bestandteile in der Regel nicht kritisch.

Gegenstand der Erfindung ist auch ein Verfahren zur Herstellung der erfindungsgemäßen Zusammensetzungen gemäß Anspruch 16, das dadurch gekennzeichnet ist, daß man das vernetzte Glykosaminglykan und die übrigen Bestandteile in dem pharmazeutischen Träger löst. Als pharmazeutischer Träger wird vorzugsweise Wasser verwendet, und zweckmäßigerweise arbeitet man unter Erwärmen und nachfolgender Abkühlung. Zum Schutz vor Oxidation kann es zweckmäßig sein, unter einer Inertgasatmosphäre, insbesondere unter Stickstoff, zu arbeiten.

Die Art, Dosis und Häufigkeit der Verabreichung der erfindungsgemäßen Zusammensetzung richtet sich insbesondere nach der Schwere der Erkrankung und dem Allgemeinzustand des Patienten, aber auch ganz allgemein nach dem Zustand und der Empfindlichkeit der Narbe der Haut. Werden die erfindungsgemäßen Zusammensetzungen in Form topisch applizierbarer Zubereitungen verabreicht, so entspricht die Verabreichung in der Regel den für derartige Zusammensetzungen üblichen Bedingungen.

Die Art der Behandlung und die Häufigkeit der Applikation richtet sich insbesondere auch nach dem individuellen Ansprechen der zu behandelnden Person. Vorzugsweise erfolgt eine Applikation von Gelen in Abständen von 1 bis 2 Monaten.

Wenn die erfindungsgemäßen Zusammensetzungen in Form injizierbarer Gele intraläsional appliziert werden, was in erster Linie der Fall ist, so geschieht dies vorzugsweise durch Injektion mit Hilfe feiner Kanülen und mit druckfesten Spritzen. Die erfindungsgemäßen Gele können aber auch mit Hilfe von Druckluftgeräten percutan eingeschossen werden; hierfür können Druckluftgeräte verwendet werden, wie sie in der Medizin für eine derartige Applikation bekannt sind.

In einer besonders bevorzugten erfindungsgemäßen Ausführungsform enthält die pharmazeutische Zubereitung in Form eines injizierbaren Gels vernetztes Heparin als vernetztes Glykosaminglykan. Während z.B. die Injektion von unvernetztem Heparin nicht in Frage kommt, weil Heparin die Blutgerinnung hemmt und außerdem rasch über das Gefäßsystem aus der Läsion abtransportiert wird, und dann, ähnlich wie die Corticoide, systemisch wirkt, verliert das Heparin in der erfindungsgemäßen vernetzten Form die blutgerinnungshemmenden Eigenschaften und kann nicht über Blut- und Lymphbahnen abtransportiert werden. Es bleibt deshalb über Wochen und Monate nach Injektion am Ort der Applikation im Keloidgewebe wirksam. Der Abbau erfolgt vornehmlich über die Phagozytose durch spezielle Zellpopulationen.

Unter Verwendung der erfindungsgemäßen Zusammensetzungen, die vernetzte Glykosaminglykane enthalten, treten auch keine lokalen Überreaktionen und keine systemischen Wirkungen auf, wie dies bei den Corticoiden der Fall ist. Ein weiterer Vorteil ist die biologische Abbaubarkeit im Organismus. So kann z.B. die intraläsionale Applikation durch Injektion in Abständen von 4 bis 8 Wochen wiederholt werden.

Ein Vorteil der bevorzugten erfindungsgemäßen Zubereitungen in Form injizierbarer Gele und deren intraläsionale Applikation besteht auch darin, daß nach Abheilung der Injektionsstellen keinerlei hygienische Zusatzmaßnahmen erforderlich sind. Es können alle Körperregionen gleichermaßen behandelt werden, und die Beweglichkeit der Patienten wird nicht durch Verbände eingeschränkt. Durch die Behandlung mit den erfindungsgemäßen Zubereitungen kann auch ein Auftreten oder Wiederauftreten von Keloiden verhindert werden, was ihre präventive Wirkung zeigt.

Gegenstand der Erfindung ist deshalb auch die Verwendung vernetzter Glykosaminglykane zur Herstellung einer pharmazeutischen Zusammensetzung zur Wund-, Narben- und Keloidbehandlung. Als vernetztes Glykosaminglykan werden dabei vorzugsweise die vorstehend bevorzugt genannten vernetzten Glykosaminglykane verwendet.

Eine besondere Anwendungsform zur Prävention von Keloiden oder kontrakten Narben ist die Applikation von wasserfreien Vorstufen der vernetzten Glykosaminglykane (z.B. als Lyophilisat) in Form eines Wundpuders in frischen Wunden. Das Puder wird dabei vor dem Wundverschluß in die offene Wunde oder Wundhöhle gestreut. Dann erfolgt der Wundverschluß durch Naht, durch Klammern o.ä.. Das Puder nimmt in der Wunde Wasser aus dem Gewebe auf und entspricht dann der erfindungsgemäßen Zubereitung in Form eines Gels bzw. stellt selbst eine erfindungsgemäße Zubereitung dar.

Puder- oder Gelform können zur Verhinderung von unerwünschten Verwachsungen auch in große Körperhöhlen eingebracht werden, z.B. in die Bauch- oder Brusthöhle, während eines chirurgischen Eingriffes am Darm oder an der Lunge, in den Herzbeutel, oder nach chirugischen Eingriffen über liegende Drainagen. Bei entzündlichen Ergüssen in große Körperhöhlen kann die erfindungsgemäße Zubereitung auch nach Punktion und Entleerung des Ergusses über die liegende Kanüle eingebracht werden.

Auch in von außen zugänglichen Höhlen und Gänge des Körpers kann die erfindungsgemäße Zubereitung eingebracht werden, z.B. in die Nasenhaupt- und -nebenhöhlen bzw. Nasengänge oder in die Tränenkanäle zur Verhinderung narbiger Verwachsungen, eventuell auch auf einem geeigneten Träger (z.B. Tampon).

Die nachfolgenden Beispiele sollen die Erfindung näher erläutern, ohne sie darauf zu beschränken.

### Beispiel 1

Herstellung eines injizierbaren Gels aus den folgenden Bestandteilen:

| Bestandteil | Menge |
|---|---|
| vernetzte Hyaluronsäure ("Hylagel" der Fa. Biomatrix, NJ, USA) | 0,004 g |
| Lidocainhydrochlorid | 0,02 g |
| Wasser, gereinigt (DAB 9) auf | 1,0 g |

Die Bestandteile werden unter Stickstoffatmosphäre unter Rühren und kurzem Erwärmen gelöst; nach dem Abkühlen wird ein farbloses, klares Gel erhalten; pH-Wert: 7,00 ± 0,1.

Das Gel wird in druckfeste Durchstichampullen eingefüllt und verschlossen. Danach wird eine Hitzesterilisation durchgeführt und das Gel unter Lichtschutz aufgehoben.

### Anwendungsbeispiel 1

Es wird die Behandlung eines ca. 3 cm x 5 cm großen dunkelroten erhabenen Keloids beschrieben, das bei einer 30-jährigen Frau am Rücken nach einer tangentialen Schnittverletzung durch eine zerbrochene Glasscheibe bestand.

Die Patientin klagte über Juckreiz im Bereich des Keloids. Das Keloid wurde im Abstand von 4 bis 8 Wochen insgesamt vier Mal mit vernetzter Hyaluronsäure (Hylon) durch Injektion infiltriert. Bereits wenige Stunden nach der ersten Injektion verschwand der Juckreiz. Innerhalb von zwei Wochen blaßte das Keloid deutlich ab und nach vier Wochen war bereits eine Abflachung zu erkennen. Nach ca. 6 Monaten war eine blasse, nur gering erhabene Narbe

### Anwendungsbeispiel 2

Es wird die Behandlung eines Keloids in den Unterbrustfalten (rechts und links) beschrieben, das bei einer Patientin nach operativer Brustkorrektur auftrat.

Die Keloide wurden mehrfach mit konservativen Methoden (Externa) behandelt und wiederholt ausgeschnitten. Nach der letzten Ausschneidung wurde während der Operation vernetzte Hyaluronsäure in die Wundränder rechts injiziert. Dann wurde seitengleich vernäht. Die Fäden wurden rechts und links nach zwei Wochen entfernt. Nach vier Wochen war die unbehandelte Narbe links deutlich mehr erhaben und mehr gerötet als die rechte mit Hyaluronsäure behandelte Narbe. Außerdem konnte das Wiederauftreten eines Keloids rechts erfolgreich verhindert werden. Dies zeigt auch die präventive Wirkung der erfindungsgemäßen pharmazeutischen Zubereitung.

## Patentansprüche

1. Pharmazeutische Zusammensetzung zur nicht-topischen Wund-, Narben- und Keloidbehandlung, enthaltend vernetzte Glykosaminglykane und übliche pharmazeutische Hilfs- und/oder Trägerstoffe.

2. Zusammensetzung nach Anspruch 1, dadurch gekennzeichnet, daß die vernetzten Glykosaminglykane durch chemische Vernetzung, Chelatbildung, Komplexbildung und/oder Salzbildung vernetzte Glykosaminglykane sind.

3. Zusammensetzung nach Anspruch 1 oder 2, dadurch gekennzeichnet, daß sie ein chemisch vernetztes Glykosaminglykan enthält.

4. Zusammensetzung nach einem der Ansprüche 1 bis 3, dadurch gekennzeichnet, daß sie Gelbildner, Mittel zur pH-Wert-Einstellung, Stabilisierungsmittel, Antioxidantien, Lösungsvermittler, penetrationsfördernde Mittel und/oder Konservierungsmittel enthält.

5. Zusammensetzung nach einem der Ansprüche 1 bis 4, dadurch gekennzeichnet, daß sie als Gel vorliegt.

6. Zusammensetzung nach Anspruch 5, dadurch gekennzeichnet, daß sie als injizierbares Gel vorliegt.

7. Zusammensetzung nach Anspruch 5 oder 6, dadurch gekennzeichnet, daß der Wassergehalt des Gels 80 bis 99 Gew.-% beträgt.

8. Zusammensetzung nach einem der Ansprüche 1 bis 7, dadurch gekennzeichnet, daß sie das vernetzte Glykosaminglykan in einer Menge von 0,1 bis 20 Gew.-% enthält.

9. Zusammensetzung nach Anspruch 8, dadurch gekennzeichnet, daß sie das vernetzte Glykosaminglykan in einer Menge von 0,5 bis 10 Gew.-%, insbesondere in einer Menge von 1 bis 5 Gew.-%, enthält.

10. Zusammensetzung nach einem der Ansprüche 1 bis 9, dadurch gekennzeichnet, daß man als vernetztes Glykosaminglykan ein solches auf der Basis eines Glykosaminglykans oder von zwei oder mehreren verschiedenen Glykosaminglykanen einsetzt.

11. Zusammensetzung nach Anspruch 10, dadurch gekennzeichnet, daß man natürliche oder synthetische Glykosaminglykane und/oder damit chemisch verwandte Substanzen einsetzt.

12. Zusammensetzung nach Anspruch 11, dadurch gekennzeichnet, daß man als natürliche Glykosaminglykane solche einsetzt, die in menschlichem Bindegewebe vorkommen.

13. Zusammensetzung nach Anspruch 11 oder 12, dadurch gekennzeichnet, daß man als Glykosaminglykan Hyaluronsäure, Heparin, Heparinsulfat und/oder Chondroitinsulfat einsetzt.

14. Zusammensetzung nach Anspruch 11, dadurch gekennzeichnet, daß man als synthetische Glykosaminglykane sulfatierte Polysaccharide einsetzt.

15. Zusammensetzung nach Anspruch 11, dadurch gekennzeichnet, daß man als chemisch verwandte Substanzen solche biologischen Ursprungs einsetzt, insbesondere Chitosamin und Chitosan.

16. Verfahren zur Herstellung einer Zusammensetzung nach einem der Ansprüche 1 bis 15, dadurch gekennzeichnet, daß man das vernetzte Glykosaminglykan und die übrigen Bestandteile in dem physikalischen Träger, vorzugsweise in Wasser, löst, gegebenenfalls unter Erwärmen und nachfolgendem Abkühlen.

17. Verwendung eines vernetzten Glykosaminglykans zur Herstellung einer pharmazeutischen Zusammensetzung zur Wund-, Narben- und Keloidbehandlung.

18. Verwendung nach Anspruch 17, dadurch gekennzeichnet, daß man als vernetztes Glykosaminglykan ein solches gemäß einem der Ansprüche 2, 3 und 10 bis 15 verwendet.

19. Verwendung nach Anspruch 17 oder 18 zur Herstellung einer intraläsional applizierbaren pharmazeutischen Zusammensetzung zur Hemmung der Narben- und Keloidbildung.

## Claims

1. Pharmaceutical composition for non-topical wound, scar and keloid treatment containing cross-linked glycosaminoglycans and conventional pharmaceutical auxiliary and/or carrier substances.

2. Composition as claimed in claim 1, wherein the cross-linked glycosaminoglycans are glycosaminoglycans cross-linked by chemical cross-linking, chelate formation, complex formation and/or salt formation.

3. Composition as claimed in claim 1 or 2, wherein it contains a chemically cross-linked glycosaminoglycan.

4. Composition as claimed in one of the claims 1 to 3, wherein it contains gel formers, agents to set the pH value, stabilizers, antioxidants, solubilizers, agents that promote penetration and/or preservatives.

5. Composition as claimed in one of the claims 1 to 4, wherein it is present as a gel.

6. Composition as claimed in claim 5, wherein it is present as an injectable gel.

7. Composition as claimed in claim 5 or 6, wherein the water content of the gel is 80 to 99 % by weight.

8. Composition as claimed in one of the claims 1 to 7, wherein it contains the cross-linked glycosaminoglycan in an amount of 0.1 to 20 % by weight.

9. Composition as claimed in claim 8, wherein it contains the cross-linked glycosaminoglycan in an amount of 0.5 to 10 % by weight, in particular in an amount of 1 to 5 % by weight.

10. Composition as claimed in one of the claims 1 to 9, wherein a cross-linked glycosaminoglycan based on one glycosaminoglycan or on two or several different glycosaminoglycans is used as the cross-linked glycosaminoglycan.

11. Composition as claimed in claim 10, wherein natural or synthetic glycosaminoglycans and/or substances that are chemically related thereto are used.

12. Composition as claimed in claim 11, wherein glycosaminoglycans that occur in human connective tissue are used as the natural glycosaminoglycans.

13. Composition as claimed in claim 11 or 12, wherein hyaluronic acid, heparin, heparin sulfate and/or chondroitin sulfate are used as the glycosaminoglycan.

14. Composition as claimed in claim 11, wherein sulfated polysaccharides are used as synthetic glycosaminoglycans.

15. Composition as claimed in claim 11, wherein substances of biological origin and in particular chitosamine and chitosan are used as the chemically related substances.

16. Process for the production of a composition as claimed in one of the claims 1 to 15, wherein the cross-linked glycosaminoglycan and the other components are dissolved in the physical carrier, preferably in water, if desired, while heating and subsequently cooling.

17. Use of a cross-linked glycosaminoglycan for the production of a pharmaceutical composition for wound, scar and keloid treatment.

18. Use as claimed in claim 17, wherein a glycosaminoglycan as claimed in one of the claims 2, 3 and 10 to 15 is used as the cross-linked glycosaminoglycan.

19. Use as claimed in claim 17 or 18 for the production of a pharmaceutical composition that can be applied intralesionally to inhibit scar and keloid formation.

## Revendications

1. Composition pharmaceutique pour le traitement non topique de plaies, de cicatrices et de chéloïdes, contenant des glycosaminoglycanes réticulés et des agents auxiliaires et/ou supports pharmaceutiques classiques.

2. Composition selon la revendication 1, chactérisée en ce que les glycosaminoglycanes réticulés sont des glycosaminoglycanes réticulés par réticulation chimique, chélatation, complexation et/ou formation de sels.

3. Composition selon la revendication 1 ou 2, caractérisée en ce qu'elle contient un glycosaminoglycane réticulé par voie chimique.

4. Composition selon l'une des revendications 1 à 3, caractérisée en ce qu'elle contient des agents gélifiants, des agents pour le réglage du pH, des stabilisants, des antioxydants, des agents de solubilisation, des agents favorisant la pénétration et/ou des conservateurs.

5. Composition selon l'une des revendications 1 à 4, caractérisée en ce qu'elle se trouve sous forme de gel.

6. Composition selon la revendication 5, caractérisée en ce qu'elle se trouve sous forme de gel injectable.

7. Composition selon la revendication 5 ou 6, caractérisée en ce que la teneur en eau du gel est de 80 à 99 % en masse.

8. Composition selon l'une des revendications 1 à 7, caractérisée en ce qu'elle contient le glycosaminoglycane réticulé en une quantité de 0,1 à 20 % en masse.

9. Composition selon la revendication 8, caractérisée en ce qu'elle contient le glycosaminoglycane réticulé en une quantité de 0,5 à 10 % en masse, en particulier en une quantité de 1 à 5 % en masse.

10. Composition selon l'une des revendications 1 à 9, caractérisée en ce que l'on utilise comme glycosaminoglycane réticulé un tel produit à base d'un glycosaminoglycane ou de deux ou plusieurs glycosaminoglycanes différents.

11. Composition selon la revendication 10, caractérisée en ce que l'on utilise des glycosaminoglycanes naturels ou synthétiques et/ou des substances qui leur sont chimiquement apparentées.

12. Composition selon la revendication 11, caractérisée en ce que l'on utilise comme glycosaminoglycanes naturels des glycosaminoglycanes se trouvant dans le tissu conjonctif humain.

13. Composition selon la revendication 11 ou 12, caractérisée en ce que l'on utilise comme glycosaminoglycane de l'acide hyaluronique, de l'héparine, du sulfate d'héparine et/ou du sulfate de chondroïtine.

14. Composition selon la revendication 11, caractérisée en ce que l'on utilise comme glycosaminoglycanes synthétiques des polysaccharides sulfatés.

15. Composition selon la revendication 11, caractérisée en ce que l'on utilise comme substances chimiquement apparentées des substances d'origine biologique, en particulier une chitosamine et un chitosane.

16. Procédé de préparation d'une composition selon l'une des revendications 1 à 15, caractérisé en ce que l'on dissout le glycosaminoglycane réticulé et les autres constituants dans le support physique, de préférence dans de l'eau, éventuellement en chauffant et en refroidissant ensuite.

17. Utilisation d'un glycosaminoglycane réticulé pour la préparation d'une composition pharmaceutique destinée au traitement des plaies, des cicatrices et des chéloïdes.

18. Utilisation selon la revendication 17, caractérisée en ce que l'on utilise comme glycosaminoglycane réticulé un glycosaminoglycane selon l'une des revendications 2, 3 et 10 à 15.

19. Utilisation selon la revendication 17 ou 18 pour la préparation d'une composition pharmaceutique à administrer à l'intérieur de la lésion pour empêcher la formation de cicatrices et de chéloïdes.
